# EUROPÄISCHE PATENTSCHRIFT

(11) **EP 3 530 314 B1**
(45) Veröffentlichungstag und Bekanntmachung des Hinweises auf die Patenterteilung: **29.07.2020**
(21) Anmeldenummer: 18157921.0
(22) Anmeldetag: 21.02.2018
(51) Int. Cl.: A61N 1/05, B21C 1/00, B23P 11/00, B21C 37/06, A61B 5/042

(54) **HERSTELLUNGSVERFAHREN FÜR EINE RINGELEKTRODE**
METHOD OF MANUFACTURING A RING ELECTRODE
PROCÉDÉ DE FABRICATION D'UNE ÉLECTRODE ANNULAIRE

(43) Veröffentlichungstag der Anmeldung: 28.08.2019
(73) Patentinhaber: Heraeus Deutschland GmbH & Co. KG, 63450 Hanau (DE)
(72) Erfinder: KEITEL, Oliver, 63450 Hanau (DE); KRENZER, Jörg, 63450 Hanau (DE); LE, Hoang-Minh, 63450 Hanau (DE); LEITOLD, Christiane, 63450 Hanau (DE)
(74) Vertreter: Maiwald Patent- und Rechtsanwaltsgesellschaft mbH

(56) Entgegenhaltungen:
- EP-A1- 3 170 574
- EP-A2- 0 962 267
- DE-A1-102012 013 338
- US-A1- 2014 200 640

## Beschreibung

### TECHNISCHES GEBIET

Die Erfindung betrifft ein Herstellungsverfahren für eine Ringelektrode, eine entsprechende Ringelektrode, ein Elektrodensystem umfassend eine solche Ringelektrode zur Verwendung dieser Ringelektrode oder dieses Elektrodensystems in einem Herzschrittmacher und/oder zur Neurostimulation. Die Ringelektrode ist generell zum Einsatz als oder in einer aktiven implantierbaren medizinischen Vorrichtung gedacht, kann aber auch anderweitig verwendet werden. Sie kann zur Signalerfassung und/oder zur Stimulation verwendet werden.

### HINTERGRUND DER ERFINDUNG

Die typischerweise sehr kleine Bauteilgröße einer Ringelektrode für eine aktive implantierbare medizinische Vorrichtung und die noch kleineren Abmessungen ihrer Teilmerkmale erfordern sehr teure und aufwändige Herstellungsanlagen und Herstellungsverfahren mit vielen einzelnen Arbeitsschritten. Konventionell werden Ringelektroden durch zerspanende Bearbeitung wie Drehen aus einem Stabmaterial hergestellt und das überschüssige Material im Inneren des Rings zum Beispiel durch Funkenerosion ausgeräumt. Die Ringelektroden werden häufig aus Edelmetall, wie zum Beispiel aus Platinlegierungen gefertigt, sodass die zerspanende Bearbeitung und das Ausräumen des überschüssigen Materials zu erheblichen Edelmetallverlusten und Kostennachteilen führen.

EP 0 962 367 A2 beschreibt ein Verfahren zur Herstellung von Verbund-Rohren aus Metall. DE 10 2012 013 338 A1 zeigt ein Verfahren zur Herstellung einer Elektrodenstruktur. EP 3 170 574 A1 zeigt ein Verfahren zur Herstellung einer Hülse zum Aufbau einer Ringelektrode. US 2014/0200640 A1 zeigt eine Kabelleiterarmatur für eine implantierbare Leitung.

### ZUSAMMENFASSUNG DER ERFINDUNG

Es ist daher eine Aufgabe der Erfindung unter anderem ein Herstellungsverfahren für eine Ringelektrode und eine solche Ringelektrode bereitzustellen, welche weniger kostenintensiv sind.

Diese Aufgabe wird durch ein Herstellungsverfahren für eine Ringelektrode, eine entsprechende Ringelektrode, ein Elektrodensystem umfassend eine solche Ringelektrode nach den Merkmalen der unabhängigen Ansprüche gelöst. Vorteilhafte Ausführungsformen und Weiterbildungen sind den Unteransprüchen und der nachfolgenden Beschreibung zu entnehmen.

Es wird vorgeschlagen, ein Herstellungsverfahren für eine Ringelektrode bereitzustellen, welches die folgenden Schritte umfasst (nicht notwendigerweise in dieser Reihenfolge):
- Bereitstellen eines Außenelements, welches ein Außenrohr umfasst,
- Bereitstellen eines ersten Innenelements, welches ein erstes Innenrohr mit einem ersten Kern aus einem Opfermaterial umfasst,
- Bereitstellen eines zweiten Innenelements, welches einen zweiten Kern aus einem Opfermaterial umfasst,
- Bilden eines Verbundrohres durch Anordnen des ersten Innenelements und des zweiten Innenelements innerhalb des Außenelements, wobei das erste Innenelement und das zweite Innenelement exzentrisch zueinander angeordnet sind,
- Ziehen des Verbundrohres in eine Längsrichtung des Verbundrohres,
- Abtrennen einer Verbundrohrscheibe von dem Verbundrohr,
- Entfernen des Opfermaterials des ersten Kerns, und
- Entfernen des Opfermaterials des zweiten Kerns, um eine Kontaktierungsöffnung in der Ringelektrode zu erhalten.

Ein Vorteil der Erfindung liegt darin, dass die Ringelektrode nicht aus dem Vollen, wie zum Beispiel aus einem Stabmaterial hergestellt wird, sondern direkt aus hohlen Rohren. Auf diese Weise kann auf eine zerspanende oder abtragende Bearbeitung der Außendurchmesser der Rohre verzichtet werden und es wird deutlich weniger Edelmetall im Inneren der Ringelektrode eingesetzt und verloren, da die Rohre keinen Edelmetallkern aufweisen, der ausgeräumt werden muss. Damit entfallen nicht nur die Kosten und der Aufwand für die zerspanende Bearbeitung und das Ausräumen, sondern auch die Kosten des Edelmetalls und der Edelmetallverluste.

Die Kontaktierungsöffnung in der Ringelektrode kann zur elektrischen und/oder zur mechanischen Kontaktierung mit einem Leiterelement dienen. Die Kontaktierungsöffnung kann damit als elektrisches Verbindungselement und/oder als mechanisches Befestigungselement für das Leiterelement dienen. Das Leiterelement kann ein Kabel oder ein Draht zur Kontaktierung der Ringelektrode mit einer medizinischen Vorrichtung, wie einem Herzschrittmacher sein.

Das Verbundrohr kann durch Einschieben des ersten Innenelements und des zweiten Innenelements in das Außenelement hergestellt werden. Dabei kann eine definierte Grenzfläche mit zum Beispiel einer definierten Materialbeschaffenheit zwischen dem Außenelement, dem ersten Innenelement und/oder dem zweiten Innenelement hergestellt werden. So kann zum Beispiel eine definierte Materialbeschaffenheit der Grenzfläche der Kontaktierungsöffnung für das Leiterelement geschaffen werden, sodass die Kontaktierung des Leiterelements an der Ringelektrode zum Beispiel durch Crimpen, Klemmen oder Einstecken besonders sicher und reproduzierbar sein kann.

Die exzentrische Anordnung des ersten Innenelements und des zweiten Innenelements zueinander kann so verstanden werden, dass die Mittelpunkte oder Schwerpunkte der beiden Innenelemente im Querschnitt nicht aufeinanderliegen. Das erste Innenelement und das zweite Innenelement sind also nicht konzentrisch angeordnet und bilden daher nicht die Form einer Zielscheibe. Das eine Innenelement kann das andere Innenelement zumindest teilweise überdecken und die beiden Innenelement liegen nebeneinander, sie weisen aber im Querschnitt keinen gemeinsamen Mittelpunkt oder Schwerpunkt auf. Auf diese Weise kann die Kontaktierungsöffnung so gebildet werden, dass sie im Querschnitt gesehen außerhalb des Mittelpunkts der Ringelektrode liegt.

Unter Ziehen oder Durchziehen kann ein Zugdruckumformen verstanden werden, bei dem ein Ausgangsdraht durch eine Ziehdüse, Ziehstein oder Matrize in mehreren Schritten auf einen reduzierten Durchmesser gebracht wird. Beim Ziehen des Verbundrohrs können die Außen- und Innenelemente aufeinander zufließen und Freiräume zwischen sich verkleinern und möglicherweise sogar schließen. Das erste Innenrohr kann zum Beispiel das zweite Innenelement so umfließen, dass das zweite Innenelemente nasenförmig in das erste Innenrohr hineinragt.

Durch das Ziehen kann zumindest teilweise ein Formschluss und/oder ein Kraftschluss zwischen den Einzelkomponenten des Verbundrohres erreicht werden, sodass eine Endgeometrie der Ringelektrode nach dem vorliegenden Herstellungsverfahren beständig ist. Das kann so verstanden werden, dass die Einzelkomponenten des Verbundrohres gegenüber durch gegenseitige mechanische Blockierung und/oder Reibung halten. Durch das Ziehen kann zumindest teilweise auch ein Stoffschluss zum Beispiel durch Kaltverschweißen der Einzelkomponenten des Verbundrohres erreicht werden. Das kann so verstanden werden, dass die Einzelkomponenten des Verbundrohres gegenüber durch chemische bzw. atomare Verbindung halten.

In einer Ausführungsform sind das Außenelement und das erste Innenelement konzentrisch zueinander angeordnet. Das kann so verstanden werden, dass die Mittelpunkte oder Schwerpunkte des Außenelements und des ersten Innenelements im Querschnitt aufeinanderliegen. Auf diese Weise kann eine zylindrische

Hauptöffnung der Ringelektrode gebildet werden.

In einer Ausführungsform ist der Durchmesser des ersten Innenelements größer als der Durchmesser des zweiten Innenelements. Vorzugsweise ist der Durchmesser des ersten Innenelements mehr als doppelt so groß wie der Durchmesser des Innenelements. Weiter bevorzugt ist der Durchmesser des ersten Innenelements mehr als dreimal so groß wie der Durchmesser des zweiten Innenelements. Auf diese Weise ist die durch das erste Innenelement gebildete Hauptöffnung der Ringelektrode deutlich größer als die durch das zweite Innenelement gebildete Kontaktierungsöffnung.

In einer Ausführungsform umfasst das Entfernen des Opfermaterials des ersten Kerns ein Beizen oder Ätzen. In einer Ausführungsform umfasst das Entfernen des Opfermaterials des Opfermaterials des zweiten Kerns ein Beizen oder Ätzen. Das Entfernen des Opfermaterials des ersten Kerns und das Entfernen des Opfermaterials des zweiten Kerns kann durch die gleiche oder eine unterschiedliche Art des Beizens oder Ätzens geschehen. Unter Beizen kann die Behandlung der Ringelektrode oder ihrer Komponenten mittels einer Beize verstanden werden. Als Beize können aggressive Chemikalien wie Säuren oder Laugen verwendet werden. Unter Ätzen kann die Abtragung von Material der Ringelektrode oder ihrer Komponenten durch Anwendung eines Ätzmittels verstanden werden. Als Ätzmittel können chemische Stoffe zur Anwendung kommen, die das zu ätzende Material in einer chemischen Reaktion verändern (meistens oxidieren) und so in Lösung bringen. Ätzmittel können Säuren oder starke Oxidantien sein. Das Beizen oder Ätzen kann durch Ultraschall, Wärme und/oder elektrischen Strom unterstützt werden.

In einer Ausführungsform geschieht das Entfernen des Opfermaterials des ersten Kerns mithilfe einer Säure. In einer Ausführungsform geschieht das Entfernen des Opfermaterials des zweiten Kerns mithilfe einer Säure. In beiden Fällen kann, muss aber nicht dieselbe Säure verwendet werden. Die Säure kann Salpetersäure, Salzsäure, Wasserstoffperoxid und/oder ähnliches sein.

In einer Ausführungsform umfasst das zweite Innenelement ein zweites Innenrohr, welches den zweiten Kern umfasst. Das zweite Innenrohr kann beim Ziehen des Verbundrohres in Freiräume zwischen dem Außenrohr und dem ersten Innenrohr fließen. Das zweite Innenrohr und/oder das erste Innenrohr kann weichgeglüht sein, um dieses Fließen zu begünstigen.

In einer Ausführungsform umfasst das Außenrohr ein Edelmetall oder eine Edelmetalllegierung. In einer Ausführungsform umfasst das erste Innenrohr ein Edelmetall oder eine Edelmetalllegierung. In einer Ausführungsform umfasst das optionale zweite Innenrohr ein Edelmetall oder eine Edelmetalllegierung. Das Außenrohr, das erste Innenrohr und/oder das zweite Innenrohr können aus dem gleichen oder aus unterschiedlichen Materialien sein. Als Edelmetalle können Metalle verstanden werden, deren Redoxpaare ein positives Standardpotential bezüglich der Normal-Wasserstoffelektrode aufweisen. Das Edelmetall kann Platin oder ähnliches sein. Die Edelmetalllegierung kann eine Platin-Iridium-Legierung oder ähnliches sein und insbesondere eine PtIr10 Legierung.

In einer Ausführungsform ist das Opfermaterial des ersten Kerns gegenüber dem Material des ersten Innenrohrs unedler. In einer Ausführungsform ist das Opfermaterial des zweiten Kerns gegenüber dem Material des ersten und/oder zweiten Innenrohrs unedler. Als unedle Metalle oder Nichtedelmetalle können Metalle verstanden werden, deren Redoxpaare ein negatives Standardpotential bezüglich der Normal-Wasserstoffelektrode aufweisen.

In einer Ausführungsform umfasst der erste Kern aus Opfermaterial ein Nichtedelmetall oder eine Nichtedelmetalllegierung. In einer Ausführungsform umfasst der zweite Kern aus Opfermaterial ein Nichtedelmetall oder eine Nichtedelmetalllegierung. Als Nichtedelmetalllegierung kann eine Legierung aus einem oder mehreren Nichtedelmetallen oder unedlen Metallen verstanden werden. Das Opfermaterial des ersten Kerns und das Opfermaterial des zweiten Kerns können aus dem gleichen oder aus unterschiedlichen Materialien sein. Die Nichtedelmetalllegierung kann aus Kupfer, einer Nickel-Kobalt-Basislegierung oder ähnlichem sein oder dieses umfassen. Für eine bessere Formstabilität der zur erzeugenden (kleineren) Öffnung kann das Opfermaterial des zweiten Kerns härter als das Opfermaterials des ersten Kerns sein. In einer Ausführungsform ist der erste Kern aus Kupfer. In einer Ausführungsform ist der zweite Kern aus einer Nickel-Kobalt-Basislegierung. Die Nickel-Kobalt-Basislegierung kann MP35N (35% Ni, 35% Co, 20% Cr and 10% Mo) oder MP35NLT sein.

Das Außenelement, sämtliche Innenelemente und/oder sämtliche Opfermaterialien können auch aus Kunststoffen, Keramiken und oder Cermets bestehen. Die Werkstoffpaarungen können beliebig so gewählt werden, dass das Opfermaterial gegenüber dem umgebenden Innenelement leichter entfernt werden kann.

In einer Ausführungsform geschieht das Ziehen des Verbundrohres mit einem Verformungsgrad zwischen 3 und 25 % pro einzelnen Zug und vorzugsweise mit einem Verformungsgrad zwischen 3 und 17 % pro einzelnem Zug. Im Gesamtverbund nach mehreren oder allen Zügen kann der Verformungsgrad zwischen 50 und 185 % liegen. Unter Verformungsgrad oder Umformgrad kann das logarithmische Verhältnis aus der Länge einer Probe nach der Verformung zu einer Länge der Probe vor der Verformung verstanden werden.

In einer Ausführungsform wird das Außenrohr und/oder eines oder sämtliche der Innenrohre vor dem Ziehen weichgeglüht, um ein Fließen der Einzelrohre in Freiräume zwischen den Einzelrohren zu begünstigen.

In einer Ausführungsform umfasst das Herstellungsverfahren nach dem Entfernen der Opfermaterialien ein Schneiden des Verbundrohres in Ringe. Das Schneiden kann berührungslos geschehen, zum Beispiel durch Drahterodieren. Zum Schneiden kann das Verbundrohr mit einer Klemmvorichtung fixiert und zum Beispiel auf einer Leiste befestigt werden.

In einer Ausführungsform umfasst das Herstellungsverfahren nach dem Entfernen der Opfermaterialien und entweder vor oder nach dem Schneiden des Verbundrohres in Ringe eine weitere Bearbeitung, die in einem Längsschnitt durch die Ringelektrode gesehen das zweite Innenelement gegenüber dem Außenelement und/oder dem ersten Innenelement in der Länge reduziert, sodass sich das zweite Innenelement in dem Längsschnitt nicht entlang der gesamten Länge des Außenelements und/oder des ersten Innenelements erstreckt. In anderen Worten, das zweite Innenelement bzw. die Kontaktierungsöffnung bildet mindestens eine Stufe in der Ringelektrode. Dies kann durch eine mechanische Bearbeitung und/oder ein Erodierverfahren geschehen.

In einer Ausführungsform umfasst das Herstellungsverfahren vor der Entfernung der Opfermaterialien keine Wärmebehandlung und insbesondere kein Rekristallisationsglühen. Dies hat den Vorteil, dass eine Diffusion zwischen Opfermaterialien und Innenelementen vermieden werden kann. Unter Rekristallisationsglühen kann ein Glühen ohne Phasenänderung bei einer Temperatur im Rekristallisationsbereich nach einer Kaltumformung, wie zum Beispiel dem Ziehen, verstanden werden. Nach dem Entfernen der Opfermaterialien kann eine Wärmebehandlung und insbesondere ein Rekristallisationsglühen vorgesehen werden, um zum Beispiel die Duktilität der Ringelektrode zu erhöhen.

Das Außenelement und sämtliche Innenelemente können im Querschnitt beliebige Formen aufweisen und insbesondere kreisrund, oval, elliptisch, halbkreisförmig, aber auch quadratisch, rechteckig, mehreckig und ähnliches sein. Das Außenelement und sämtliche Innenelemente können voneinander unterschiedliche Querschnitte aufweisen. Vorzugsweise sind das Außenelement und sämtliche Innenelemente im Querschnitt kreisrund.

In einer Ausführungsform ist das Außenrohr und/oder eines oder sämtliche der Innenrohre ein Profilrohr. Als Profilrohr kann ein Rohr verstanden werden, welches im Querschnitt eine nicht kreisrunde Form aufweist, wie zum Beispiel eine im Querschnitt quadratische, rechteckige, halbrunde oder bogenförmige Form. In einer Ausführungsform ist das erste Innenrohr ein Profilrohr. Das Innenohr kann dabei größtenteils kreisrund sein, aber an mindestens einer Stelle eine bogenförmige Ausbuchtung aufweisen, die gestaltet ist, um das zweite Innenelement aufzunehmen. Das Profilrohr kann auch an einer weiteren Stelle eine bogenförmige Ausbuchtung für ein weiteres Innenelement aufweisen. Die Ausbuchtung des Profilrohres kann auch trapezförmig sein.

Durch das erfindungsgemäße Herstellungsverfahren können beliebige Anzahlen und Anordnungen von Öffnungen in einer Ringelektrode erzeugt werden. Durch das Entfernen des Opfermaterials des ersten Kerns kann eine Durchgangsöffnung in der Ringelektrode erzeugt werden. Durch das Entfernen des Opfermaterials des zweiten Kerns kann eine Kontaktierungsöffnung zur elektrischen und/oder mechanischen Kontaktierung erzeugt werden. Durch das Entfernen eines Opfermaterials eines optionalen dritten Kerns kann eine weitere Öffnung in der Ringelektrode erzeugt werden. In einer Ausführungsform umfasst das Herstellungsverfahren dafür weiterhin die folgenden Schritte:
- Bereitstellen eines dritten Innenelements, welches einen dritten Kern aus einem Opfermaterial umfasst,
- Bilden des Verbundrohres durch Anordnen des dritten Innenelements innerhalb des Außenelements, wobei die ersten, zweiten und dritten Innenelemente exzentrisch zueinander angeordnet sind, und
- Entfernen des Opfermaterials des dritten Kerns.

Das dritte Innenelement kann ein drittes Innenrohr aufweisen, welches den dritten Kern aus Opfermaterials umfasst. Das Opfermaterial des dritten Kerns kann wie oben beschrieben durch Beizen oder Ätzen entfernt werden. Die durch das Entfernen des dritten Kerns geschaffene weitere Öffnung der Ringelektrode kann gegenüber der durch Entfernen des zweiten Kerns geschaffene Kontaktierungsöffnung am Außenumfang des ersten Innenrohrs angeordnet sein. Die durch das Entfernen des ersten Kerns geschaffene Durchgangsöffnung der Ringelektrode kann apfelförmig ausgebildet sein, sodass die Kontaktierungsöffnung und die weitere Öffnung jeweils in den gegenüberliegenden Ausbuchtungen der apfelförmigen Durchgangsöffnung am Außenumfang der Kontaktierungsöffnung angeordnet sein können.

Es wird weiterhin vorgeschlagen, eine Ringelektrode bereitzustellen, welche ein Außenelement, ein erstes Innenelement und ein zweites Innenelement umfasst. Das Außenelement umfasst ein Außenrohr. Das erste Innenelement und das zweite Innenelement sind innerhalb des Außenelements angeordnet. Das erste Innenelement und das zweite Innenelement sind exzentrisch zueinander angeordnet, um ein Verbundrohr zu bilden. Das Außenelement, das erste Innenelement und das zweite Innenelement sind miteinander in eine Längsrichtung des Verbundrohres gezogen worden. Das erste Innenelement weist ein erstes Innenrohr auf, welches einen ersten Hohlraum umgibt, aus dem ein Opfermaterial entfernt wurde. Das zweite Innenelement umgibt einen zweiten Hohlraum, aus dem ein Opfermaterial entfernt wurde und der eine Kontaktierungsöffnung in der Ringelektrode bildet.

In einer Ausführungsform weist die Ringelektrode in einem Querschnitt gesehen eine Grenzlinie, Schnittstelle oder "Naht" zwischen dem Außenelement und dem ersten Innenelement auf. Das kann so verstanden werden, dass das Außenelement und das erste Innenelement nicht vollständig ineinander übergehen und miteinander verschmelzen, sondern unter dem Mikroskop die beiden Elemente noch als ursprünglich unterschiedliche Komponenten erkennbar sind.

Im Folgenden werden einige mögliche Abmessungen der Ringelektrode genannt. Die einzelnen Abmessungen sind unabhängig voneinander zu verstehen und bilden nicht zwingend eine gemeinsame Ausführungsform, dies ist aber möglich. Ein Außendurchmesser der Ringelektrode und damit ein Außendurchmesser des Außenelements und des Außenrohrs kann zwischen 1 und 3 mm liegen, vorzugsweise zwischen 1,3 und 2,5 mm und weiter bevorzugt zwischen 1,5 und 2,0 mm. Ein Innendurchmesser des ersten Innenelements und damit ein Innendurchmesser des ersten Innenrohrs kann zwischen 0,9 und 2,9 mm liegen, vorzugsweise zwischen 1,2 und 2,4 mm und weiter bevorzugt zwischen 1,4 und 1,9 mm. Ein Innendurchmesser der Kontaktierungsöffnung und damit ein Außendurchmesser des zweiten Kerns kann zwischen 0,10 und 0,30 mm liegen, vorzugsweise zwischen 0,15 und 0,25 mm und weiter bevorzugt zwischen 0,17 und 0,20 mm.

Es wird weiterhin vorgeschlagen, ein Elektrodensystem bereitzustellen, welches eine solche Ringelektrode und ein Leiterelement umfasst. Das Leiterelement ist mit einer Kontaktierungsöffnung in der Ringelektrode verbunden. Das Leiterelement kann ein Draht, ein Kabel oder ähnliches sein. Die Kontaktierungsöffnung in der Ringelektrode kann eine Art kleines, inneres Loch zur elektrischen und/oder zur mechanischen Kontaktierung des Leiterelements sein. Die Kontaktierungsöffnung kann daher ein Befestigungselement für das Leiterelement sein. Das Leiterelement kann durch Schweißen, insbesondere Laserschweißen oder Widerstandsschweißen, Löten, Crimpen oder ähnliches mit der Kontaktierungsöffnung bzw. dem Befestigungselement der Ringelektrode verbunden werden. Auf diese Weise wird eine besonders sichere und einfache Befestigung des Leiterelements an der Ringelektrode erreicht.

Es wird weiterhin vorgeschlagen, eine solche Ringelektrode oder ein solches Elektrodensystem, welche nach dem hier beschriebenen Herstellungsverfahren hergestellt wurden, in einem Herzschrittmacher oder zur Neurostimulation zu verwenden. Die Erfindung kann als Stimulations- oder Messelektrode für Herzschrittmacherelektroden eingesetzt werden, insbesondere für ventrikuläre, Vorhof - und linksventrikuläre Zuleitungen. Die Erfindung kann auch zur Neurostimulation zum Beispiel bei einer Rückenmarkstimulation oder einer Tiefenhirnstimulation eingesetzt werden. Des Weiteren ist ein Einsatz auf Kathetern zum Beispiel bei Elektrophysiologieanwendungen möglich, wie zum Beispiel für eine Ablation, Herzstrommessung oder ähnliches. Natürlich sind auch weitere Einsatzmöglichkeiten möglich.

### KURZE BESCHREIBUNG DER ZEICHNUNGEN

Weitere Merkmale, Vorteile und Anwendungsmöglichkeiten der vorliegenden Erfindung ergeben sich aus der nachfolgenden Beschreibung der Ausführungsbeispiele und den Figuren. In den Figuren stehen gleiche Bezugszeichen für gleiche oder ähnliche Objekte.
- **Figur 1**: zeigt ein Herstellungsverfahren für eine Ringelektrode.
- **Figur 2**: zeigt mehrere Ausführungsformen eines Schritts des Herstellungsverfahrens für eine Ringelektrode.
- **Figur 3**: zeigt mehrere Teilschritte eines weiteren Schritts des Herstellungsverfahrens für eine Ringelektrode.
- **Figur 4**: zeigt eine Ringelektrode in einer Aufsicht und beispielhafte Bemaßungen der Ringelektrode.
- **Figur 5**: zeigt beispielhafte Längsschnitte durch eine Ringelektrode.

### DETAILLIERTE BESCHREIBUNG EXEMPLARISCHER AUSFÜHRUNGSFORMEN

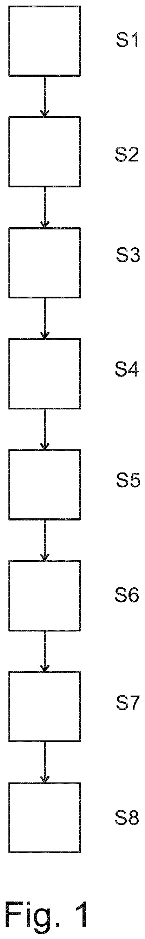

**Figur 1** zeigt ein Herstellungsverfahren für eine Ringelektrode 10. Die Ringelektrode 10 kann als aktive implantierbare medizinischen Vorrichtung zum Beispiel in einem Herzschrittmacher oder zur Neurostimulation eingesetzt werden. Sie kann zur Signalerfassung und zur Stimulation verwendet werden.

Das Herstellungsverfahren für die Ringelektrode 10 umfasst die folgenden Schritte (nicht notwendigerweise in dieser Reihenfolge):
- In einem Schritt S1,: Bereitstellen eines Außenelements 11, welches ein Außenrohr 12 umfasst.
- In einem Schritt S2,: Bereitstellen eines ersten Innenelements 13, welches ein erstes Innenrohr 14 mit einem ersten Kern 15 aus einem Opfermaterial umfasst.
- In einem Schritt S3,: Bereitstellen eines zweiten Innenelements 16, welches einen zweiten Kern 17 aus einem Opfermaterial umfasst.
- In einem Schritt S4,: Bilden eines Verbundrohres durch Anordnen des ersten Innenelements 13 und des zweiten Innenelements 16 innerhalb des Außenelements 11, wobei das erste Innenelement 13 und das zweite Innenelement 16 exzentrisch zueinander angeordnet sind.
- In einem Schritt S5,: Ziehen des Verbundrohres in eine Längsrichtung des Verbundrohres.
- In einem Schritt S6,: Abtrennen einer Verbundrohrscheibe von dem Verbundrohr,
- In einem Schritt S7,: Entfernen des Opfermaterials des ersten Kerns 15, um eine Ringelektrode 10 zu erhalten.
- In einem Schritt S8,: Entfernen des Opfermaterials des zweiten Kerns 17, um eine Kontaktierungsöffnung 2 in der Ringelektrode 10 zu erhalten.

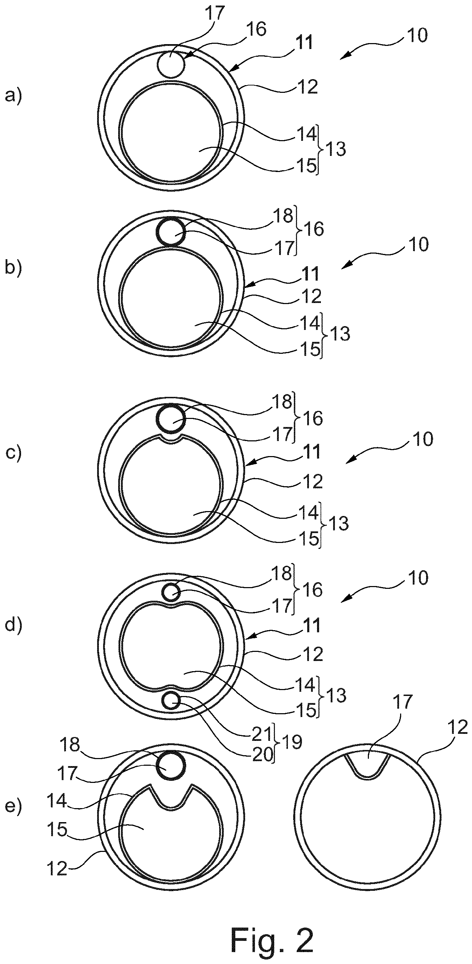

Die **Figuren 2a bis 2e** zeigen Aufsichten mehrerer Ausführungsformen einer Vorstufe der Ringelektrode 10 nach dem Schritt S4, also nach dem Bilden des Verbundrohres, aber vor dem Schritt S5, dem Ziehen des Verbundrohres. Die Vorstufe der Ringelektrode 10 umfasst ein Außenelement 11, ein erstes Innenelement 13 und ein zweites Innenelement 16.

Insbesondere in der in **Figur 2a** gezeigten Ausführungsform ist das Außenelement 11 kreisrund und umfasst ein kreisrundes Außenrohr 12. Das erste Innenelement 13 und das zweite Innenelement 16 sind ebenfalls kreisrund und liegen innerhalb des Außenelements 11 und seines Außenrohrs 12. Das erste Innenelement 13 und das zweite Innenelement 16 sind exzentrisch zueinander angeordnet, dass heißt die Mittelpunkte der beiden Innenelemente liegen nicht aufeinander. Der Durchmesser des ersten Innenelements 13 ist deutlich größer als der Durchmesser des zweiten Innenelements 16.

Das erste Innenelement 13 weist ein kreisrundes erstes Innenrohr 14 auf, welches einen ebenfalls kreisrunden ersten Hohlraum umgibt, welcher ein erstes Opfermaterial umfasst. Das zweite Innenelement 16 umgibt einen kreisrunden zweiten Hohlraum, welcher ein zweites Opfermaterial umfasst.

Das Außenrohr 12 und das erste Innenrohr 13 bestehen hier aus der Legierung PtIr10. Der erste Kern 15 besteht hier aus Kupfer. Der zweite Kern 17 besteht hier aus MP35N. Durch das Entfernen des Opfermaterials des ersten Kerns 15 kann in dem nachfolgenden Herstellungsschritt S7 eine Durchgangsöffnung 3 in der Ringelektrode 10 erzeugt werden. Der Schritt S7 kann ein Beizen mit Salpetersäure in einem Ultraschallbad bei 80°C sein. Durch das Entfernen des Opfermaterials des zweiten Kerns 17 kann in dem nachfolgenden Herstellungsschritt S8 eine Kontaktierungsöffnung 2 zur elektrischen und/oder mechanischen Kontaktierung erzeugt werden. Der Schritt S8 kann ein Beizen mit HCl und H₂O₂ im Verhältnis 3:1 für 15 Minuten in einem Ultraschallbad bei 80°C sein. Die Kontaktierungsöffnung 2 kann als elektrisches Verbindungselement und/oder als mechanisches Befestigungselement für ein Leiterelement dienen, um einen Elektrodensystem aus der Ringelektrode 10 und dem Leiterelement zu bilden.

In der in der **Figur 2b** gezeigten Ausführungsform umfasst das zweite Innenelement 16 ein zweites Innenrohr 18, welches den zweiten Kern 17 umfasst. Das zweite Innenrohr 18 kann beim Ziehen des Verbundrohres in Freiräume zwischen dem Außenrohr 12 und dem ersten Innenrohr 13 fließen. Das zweite Innenrohr 18 besteht hier ebenfalls aus PtIr10.

In den in den **Figuren 2c bis 2e** gezeigten Ausführungsformen ist das erste Innenrohr 13 ein Profilrohr. Das Innenrohr 13 ist größtenteils kreisrund, hat aber in den in den Figuren 2c und 2e gezeigten Ausführungsformen an einer Stelle eine bogenförmige (Figur 2c) bzw. trapezförmig (Figur 2e) Ausbuchtung, um das zweite Innenelement 16 aufzunehmen.

In der in der **Figur 2d** gezeigten Ausführungsform weist das Profilrohr des ersten Innenrohrs 13 noch an einer weiteren, dem zweiten Innenelement 16 gegenüberliegenden Stelle eine bogenförmige Ausbuchtung für ein weiteres, drittes Innenelement 19 auf. Das dritte Innenelement 19 liegt innerhalb des Außenelements 11 und die ersten, zweiten und dritten Innenelemente sind exzentrisch zueinander angeordnet. Das dritte Innenelement 19 umfasst ein drittes Innenrohr 21 und einen dritten Kern 20 aus einem Opfermaterial, durch dessen Entfernen eine weitere Öffnung in der Ringelektrode 10 erzeugt werden kann. In der in der Figur 2d gezeigten Ausführungsform wird durch das Entfernen des ersten Kerns 15 eine apfelförmige Durchgangsöffnung 3 der Ringelektrode 10 geschaffen, bei der die Kontaktierungsöffnung 2 und die weitere Öffnung jeweils in den gegenüberliegenden Ausbuchtungen der apfelförmigen Durchgangsöffnung 3 angeordnet sind.

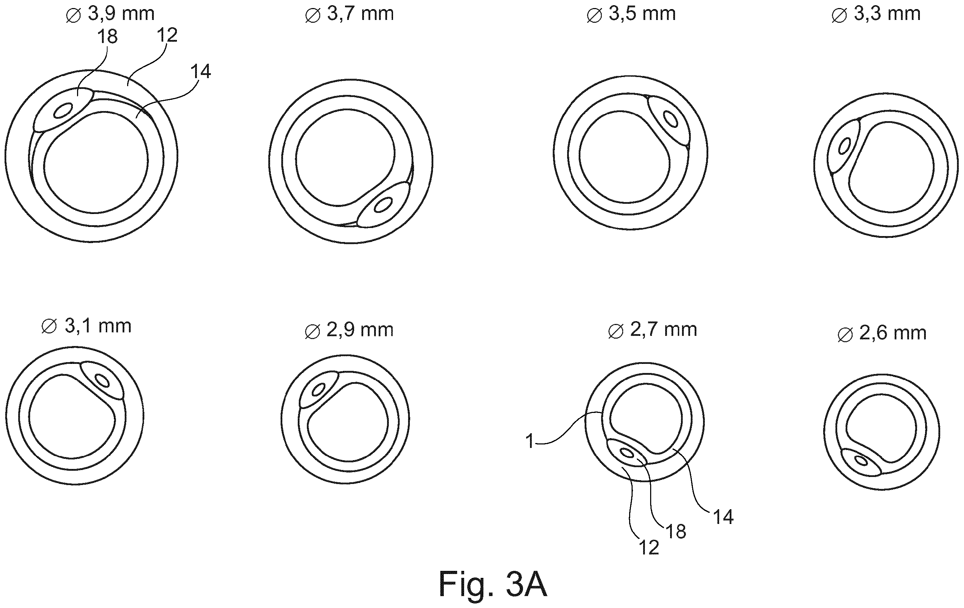

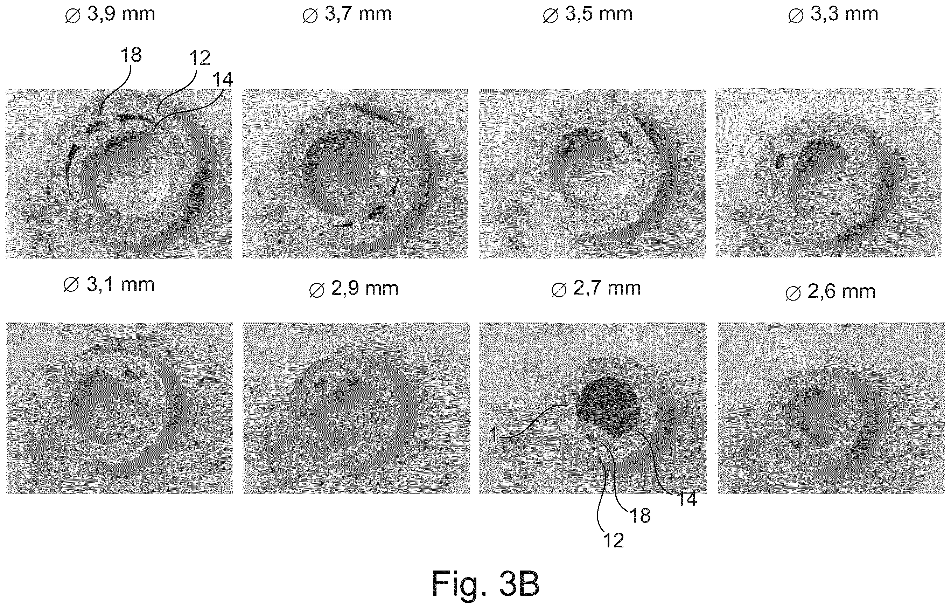

Die **Figuren 3a** **und** **3b** zeigen Aufsichten auf die Ringelektrode 10 nach mehreren Teilschritten des Ziehens des Verbundrohres in Längsrichtung als Fotographien und Zeichnungen. Das Ziehen des Verbundrohres kann gemäß folgender Ziehfolge auf die folgenden Durchmesser geschehen.

| **Ziehfolge Ø [mm]** |
|---|
| 5 |
| 4,9 |
| 4,8 |
| 4,7 |
| 4,6 |
| 4,5 |
| 4,4 |
| 4,3 |
| 4,2 |
| 4,1 |
| 4 |
| 3,9 |
| 3,8 |
| 3,7 |
| 3,6 |
| 3,5 |
| 3,4 |
| 3,3 |
| 3,2 |
| 3,1 |
| 3 |
| 2,9 |
| 2,8 |
| 2,7 |
| 2,6 |

Figur 3 zeigt die Ringelektrode 10 mit immer kleiner werdenden Durchmessern. Es ist das Verschmelzen oder ineinander Fließen des Außenrohrs 12 und des ersten Innenrohrs 14 zu erkennen. Die Freiräume oder Hohlräume zwischen diesen beiden Elementen verschwinden mehr und mehr. Lediglich eine Grenzlinie 1 bleibt zwischen dem Außenelement 11 und dem ersten Innenelement 13 sichtbar.

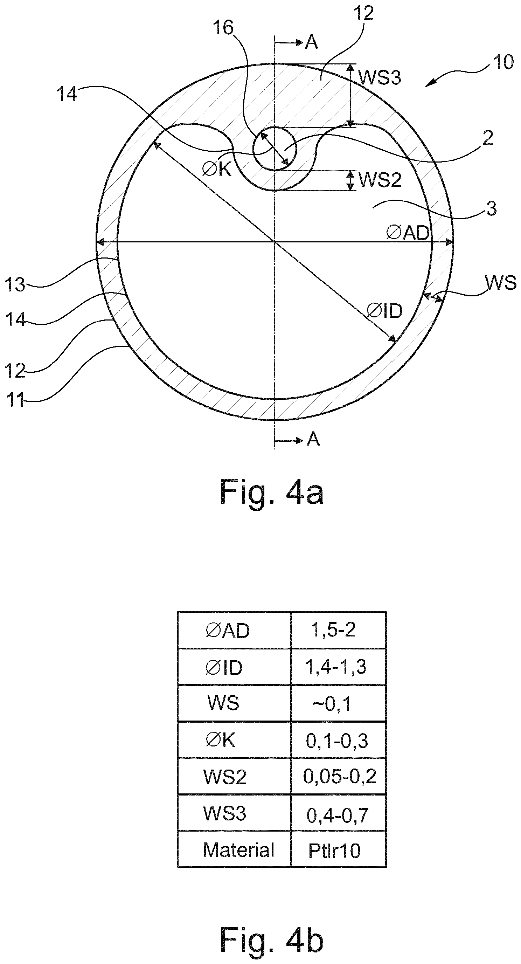

**Figur 4a** zeigt eine Ringelektrode 10 in einer Aufsicht und **Figur 4b** zeigt drei beispielhafte Bemaßungen der Ringelektrode 10 nach Figur 4a in Millimetern. Die Ringelektrode 10 umfasst ein Außenelement 11 mit einem Außenrohr 12 und ein erstes Innenelement 13 mit einem ersten Innenrohr 14. Das Außenrohr 12 und das erste Innenrohr 14 sind ineinander übergegangen. Exzentrisch zum ersten Innenrohr 14 ist ein deutlich kleineres, zweites Innenelement 16 angeordnet, welches von dem ersten Innenrohr 14 umflossen wird. Das erste Innenrohr 14 bildet eine Durchgangsöffnung 3 und das zweite Innenelement 16 eine Kontaktierungsöffnung 2 für die Ringelektrode 10. Die vom ersten Innenrohr 14 teilweise umschlossene Kontaktierungsöffnung 2 ragt nasenförmig in die Durchgangsöffnung 3 der Ringelektrode 10 hinein.

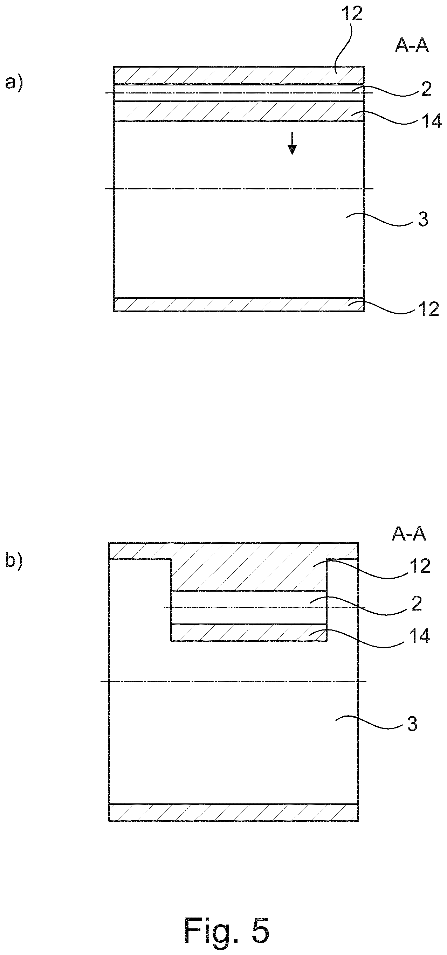

Die **Figuren 5a und 5b** zeigen beispielhafte Längsschnitte durch eine Längsrichtung der Ringelektrode 10 nach Figur 4. Figur 5a zeigt eine Ringelektrode 10 mit einem konstanten Innendurchmesser. Die Figur 5b zeigt eine Ringelektrode 10 mit einem asymmetrischen und stufenförmigen Innendurchmesser. Das zweite Innenelement 16 ist gegenüber dem Außenelement 11 in der Länge reduziert, sodass sich das zweite Innenelement 16 und damit die Kontaktierungsöffnung 2 im Längsschnitt nicht entlang der gesamten Länge des Außenelements 11 erstreckt. In der in der Figur 5b gezeigten Ringelektrode 10 endet die Kontaktierungsöffnung 2 an ihren beiden Enden innerhalb des Außenelements 11 der Ringelektrode 10, die Kontaktierungsöffnung 2 schließt also an keinem Ende bündig mit der Ringelektrode 10 ab.

Ergänzend ist darauf hinzuweisen, dass "umfassend" keine anderen Elemente oder Schritte ausschließt und "eine" oder "ein" keine Vielzahl ausschließt. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

## Patentansprüche

1. Ein Herstellungsverfahren für eine Ringelektrode (10), umfassend die folgenden Schritte:
- Bereitstellen eines Außenelements (11), welches ein Außenrohr (12) umfasst,
- Bereitstellen eines ersten Innenelements (13), welches ein erstes Innenrohr (14) mit einem ersten Kern (15) aus einem Opfermaterial umfasst,
- Bereitstellen eines zweiten Innenelements (16), welches einen zweiten Kern (17) aus einem Opfermaterial umfasst,
- Bilden eines Verbundrohres durch Anordnen des ersten Innenelements (13) und des zweiten Innenelements (16) innerhalb des Außenelements (11), wobei das erste Innenelement (13) und das zweite Innenelement (16) exzentrisch zueinander angeordnet sind,
- Ziehen des Verbundrohres in eine Längsrichtung des Verbundrohres,
- Abtrennen einer Verbundrohrscheibe von dem Verbundrohr,
- Entfernen des Opfermaterials des ersten Kerns (15), und
- Entfernen des Opfermaterials des zweiten Kerns (17), um eine Kontaktierungsöffnung (2) in der Ringelektrode (10) zu erhalten.

2. Herstellungsverfahren nach Anspruch 1, wobei das Entfernen des Opfermaterials des ersten Kerns (15) und/oder des Opfermaterials des zweiten Kerns (17) ein Beizen oder Ätzen ist.

3. Herstellungsverfahren nach einem der vorherigen Ansprüche, wobei das Entfernen des Opfermaterials des ersten Kerns (15) und/oder des Opfermaterials des zweiten Kerns (17) mithilfe einer Säure geschieht, vorzugsweise durch Salpetersäure, Salzsäure und/oder Wasserstoffperoxid.

4. Herstellungsverfahren nach einem der vorherigen Ansprüche, wobei das Opfermaterial des ersten Kerns (15) und/oder das Opfermaterial des zweiten Kerns (17) gegenüber dem Material des Außenrohrs (12) und/oder des ersten Innenrohrs (14) unedler ist.

5. Herstellungsverfahren nach einem der vorherigen Ansprüche, wobei das Außenrohr (12) und/oder das erstes Innenrohr (14) ein Edelmetall oder eine Edelmetalllegierung und vorzugsweise eine Platin-Iridium-Legierung umfasst.

6. Herstellungsverfahren nach einem der vorherigen Ansprüche, wobei der erste Kern (15) aus Opfermaterial und/oder der zweite Kern (17) aus Opfermaterial eine Nichtedelmetalllegierung und vorzugsweise Kupfer und/oder MP35N umfasst.

7. Herstellungsverfahren nach einem der vorherigen Ansprüche, wobei das Ziehen des Verbundrohres mit einem Verformungsgrad zwischen 3 und 25 % pro einzelnen Zug geschieht.

8. Herstellungsverfahren nach einem der vorherigen Ansprüche, wobei der Durchmesser des ersten Innenelements (13) größer ist als der Durchmesser des zweiten Innenelements (16).

9. Herstellungsverfahren nach einem der vorherigen Ansprüche, wobei das zweite Innenelement (16) ein zweites Innenrohr (18) umfasst, welches den zweiten Kern (17) umgibt.

10. Herstellungsverfahren nach einem der vorherigen Ansprüchen, weiterhin umfassend
- Bereitstellen eines dritten Innenelements (19), welches einen dritten Kern (20) aus einem Opfermaterial umfasst,
- Bilden des Verbundrohres durch Anordnen des dritten Innenelements (19) innerhalb des Außenelements (11), wobei die ersten, zweiten und dritten Innenelemente exzentrisch zueinander angeordnet sind, und
- Entfernen des Opfermaterials des dritten Kerns (20).

11. Herstellungsverfahren nach einem der vorherigen Ansprüche, wobei das Herstellungsverfahren kein Rekristallisationsverfahren umfasst.

12. Eine Ringelektrode (10), umfassend:
- ein Außenelement (11),
- ein erstes Innenelement (13), und
- ein zweites Innenelement (16),
wobei das Außenelement (11) ein Außenrohr (12) umfasst,
wobei das erste Innenelement (13) und das zweite Innenelement (16) innerhalb des Außenelements (11) angeordnet sind und das erste Innenelement (13) und das zweite Innenelement (16) exzentrisch zueinander angeordnet sind, um ein Verbundrohr zu bilden,
wobei das Außenelement (11), das erste Innenelement (13) und das zweite Innenelement (16) miteinander in eine Längsrichtung des Verbundrohres gezogen sind,
wobei das erste Innenelement (13) ein erstes Innenrohr (14) aufweist, welches einen ersten Hohlraum umgibt, aus dem ein Opfermaterial entfernt wurde, und
wobei das zweite Innenelement (16) einen zweiten Hohlraum umgibt, aus dem ein Opfermaterial entfernt wurde und der eine Kontaktierungsöffnung (2) in der Ringelektrode (10) bildet.

13. Ringelektrode (10) nach dem vorherigen Anspruch, wobei die Ringelektrode (10) in einem Querschnitt gesehen eine Grenzlinie (1) zwischen dem Außenelement (11) und dem ersten Innenelement (13) aufweist.

14. Ein Elektrodensystem umfassend eine Ringelektrode (10) nach einem der vorherigen Ansprüche und ein Leiterelement (20), wobei das Leiterelement (20) mit einer Kontaktierungsöffnung (2) in der Ringelektrode (10) verbunden ist.

## Claims

1. Production method for a ring electrode (10), comprising the following steps:
- providing an outer element (11) which comprises an outer tube (12),
- providing a first inner element (13) which comprises a first inner tube (14) with a first core (15) composed of a sacrificial material,
- providing a second inner element (16) which comprises a second core (17) composed of a sacrificial material,
- forming a composite tube by arranging the first inner element (13) and the second inner element (16) inside the outer element (11), wherein the first inner element (13) and the second inner element (16) are arranged eccentrically in relation to one another,
- drawing the composite tube in a longitudinal direction of the composite tube,
- separating a composite tube disc from the composite tube,
- removing the sacrificial material of the first core (15), and
- removing the sacrificial material of the second core (17) in order to obtain a contact-making opening (2) in the ring electrode (10).

2. Production method according to Claim 1, wherein the removal of the sacrificial material of the first core (15) and/or of the sacrificial material of the second core (17) is corrosion or etching.

3. Production method according to either of the preceding claims, wherein the removal of the sacrificial material of the first core (15) and/or of the sacrificial material of the second core (17) is carried out with the aid of an acid, preferably by nitric acid, hydrochloric acid and/or hydrogen peroxide.

4. Production method according to one of the preceding claims, wherein the sacrificial material of the first core (15) and/or the sacrificial material of the second core (17) is less noble than the material of the outer tube (12) and/or of the first inner tube (14).

5. Production method according to one of the preceding claims, wherein the outer tube (12) and/or the first inner tube (14) comprise/comprises a noble metal or a noble metal alloy, and preferably a platinum-iridium alloy.

6. Production method according to one of the preceding claims, wherein the first core (15) composed of sacrificial material and/or the second core (17) composed of sacrificial material comprises a non-noble metal alloy, and preferably copper and/or MP35N.

7. Production method according to one of the preceding claims, wherein the drawing of the composite tube is carried out with a degree of deformation of between 3 and 25% per individual drawing.

8. Production method according to one of the preceding claims, wherein the diameter of the first inner element (13) is greater than the diameter of the second inner element (16).

9. Production method according to one of the preceding claims, wherein the second inner element (16) comprises a second inner tube (18) which surrounds the second core (17) .

10. Production method according to one of the preceding claims, furthermore comprising
- providing a third inner element (19) which comprises a third core (20) composed of a sacrificial material,
- forming the composite tube by arranging the third inner element (19) inside the outer element (11), wherein the first, second and third inner elements are arranged eccentrically in relation to one another, and
- removing the sacrificial material of the third core (20) .

11. Production method according to one of the preceding claims, wherein the production method does not comprise a recrystallization method.

12. Ring electrode (10), comprising:
- an outer element (11),
- a first inner element (13), and
- a second inner element (16),
wherein the outer element (11) comprises an outer tube (12),
wherein the first inner element (13) and the second inner element (16) are arranged inside the outer element (11) and the first inner element (13) and the second inner element (16) are arranged eccentrically in relation to one another in order to form a composite tube,
wherein the outer element (11), the first inner element (13) and the second inner element (16) are drawn together in a longitudinal direction of the composite tube, wherein the first inner element (13) has a first inner tube (14) which surrounds a first cavity from which a sacrificial material has been removed, and
wherein the second inner element (16) surrounds a second cavity from which a sacrificial material has been removed and which forms a contact-making opening (2) in the ring electrode (10).

13. Ring electrode (10) according to the preceding claim, wherein the ring electrode (10), as seen in a cross section, has a boundary line (1) between the outer element (11) and the first inner element (13).

14. Electrode system comprising a ring electrode (10) according to one of the preceding claims and a conductor element (20), wherein the conductor element (20) is connected to a contact-making opening (2) in the ring electrode (10).

## Revendications

1. Procédé de fabrication d'une électrode annulaire (10), comprenant les étapes suivantes :
- fourniture d'un élément extérieur (11) comprenant un tube extérieur (12),
- fourniture d'un premier élément intérieur (13) qui comprend un premier tube intérieur (14) comportant un premier noyau (15) en matériau sacrificiel,
- fourniture d'un deuxième élément intérieur (16) comprenant un deuxième noyau (17) en matériau sacrificiel,
- formation d'un tube composite en disposant le premier élément intérieur (13) et le deuxième élément intérieur (16) à l'intérieur de l'élément extérieur (11), dans lequel le premier élément intérieur (13) et le deuxième élément intérieur (16) sont disposés de manière excentrique l'un par rapport à l'autre,
- étirage du tube composite dans la direction longitudinale du tube composite,
- séparation d'un disque de tube composite du tube composite,
- enlèvement du matériau sacrificiel du premier noyau (15), et
- enlèvement du matériau sacrificiel du deuxième noyau (17) pour obtenir une ouverture de contact (2) dans l'électrode annulaire (10).

2. Procédé de fabrication selon la revendication 1, dans lequel l'enlèvement du matériau sacrificiel du premier noyau (15) et/ou du matériau sacrificiel du deuxième noyau (17) est un décapage ou une gravure.

3. Procédé de fabrication selon l'une des revendications précédentes, dans lequel l'enlèvement du matériau sacrificiel du premier noyau (15) et/ou du matériau sacrificiel du deuxième noyau (17) s'effectue à l'aide d'un acide, de préférence l'acide nitrique, l'acide chlorhydrique et/ou le peroxyde d'hydrogène.

4. Procédé de fabrication selon l'une des revendications précédentes, dans lequel le matériau sacrificiel du premier noyau (15) et/ou le matériau sacrificiel du deuxième noyau (17) est moins noble que le matériau du tube extérieur (12) et/ou du premier tube intérieur (14).

5. Procédé de fabrication selon l'une des revendications précédentes, dans lequel le tube extérieur (12) et/ou le premier tube intérieur (14) comprend un métal noble ou un alliage de métal noble et, de préférence, un alliage de platine-iridium.

6. Procédé de fabrication selon l'une des revendications précédentes, dans lequel le premier noyau (15) en matériau sacrificiel et/ou le deuxième noyau (17) en matériau sacrificiel comprend un alliage de métal non noble et, de préférence, du cuivre et/ou du MP35N.

7. Procédé de fabrication selon l'une des revendications précédentes, dans lequel l'étirage du tube composite s'effectue avec un degré de déformation compris entre 3 et 25 % par étirage individuel.

8. Procédé de fabrication selon l'une des revendications précédentes, dans lequel le diamètre du premier élément intérieur (13) est supérieur au diamètre du deuxième élément intérieur (16).

9. Procédé de fabrication selon l'une des revendications précédentes, dans lequel le deuxième élément intérieur (16) comprend un deuxième tube intérieur (18) qui entoure le deuxième noyau (17).

10. Procédé de fabrication selon l'une des revendications précédentes, comprenant en outre
- la fourniture d'un troisième élément intérieur (19) comprenant un troisième noyau (20) en matériau sacrificiel,
- la formation du tube composite en disposant le troisième élément intérieur (19) à l'intérieur de l'élément extérieur (11), dans lequel les premier, deuxième et troisième éléments intérieurs sont disposés de manière excentrique les uns par rapport aux autres, et
- l'enlèvement du matériau sacrificiel du troisième noyau (20) .

11. Procédé de fabrication selon l'une des revendications précédentes, dans lequel le procédé de fabrication ne comprend pas de procédé de recristallisation.

12. Électrode annulaire (10), comprenant :
- un élément extérieur (11),
- un premier élément intérieur (13), et
- un deuxième élément intérieur (16),
dans lequel l'élément extérieur (11) comprend un tube extérieur (12),
dans laquelle le premier élément intérieur (13) et le deuxième élément intérieur (16) sont disposés à l'intérieur de l'élément extérieur (11) et le premier élément intérieur (13) et le deuxième élément intérieur (16) sont disposés de manière excentrique l'un par rapport à l'autre afin de former un tube composite, dans laquelle l'élément extérieur (11), le premier élément intérieur (13) et le deuxième élément intérieur (16) sont étirés ensemble dans une direction longitudinale du tube composite,
dans laquelle le premier élément intérieur (13) comprend un premier tube intérieur (14) qui entoure une première cavité de laquelle un matériau sacrificiel a été enlevé, et
dans laquelle le deuxième élément intérieur (16) entoure une deuxième cavité de laquelle un matériau sacrificiel a été enlevé et qui forme une ouverture de contact (2) dans l'électrode annulaire (10).

13. Électrode annulaire (10) selon la revendication précédente, dans laquelle l'électrode annulaire (10), telle que vue en coupe transversale, présente une ligne de délimitation (1) entre l'élément extérieur (11) et le premier élément intérieur (13).

14. Système d'électrode comprenant une électrode annulaire (10) selon l'une des revendications précédentes et un élément conducteur (20), dans lequel l'élément conducteur (20) est relié à une ouverture de contact (2) dans l'électrode annulaire (10).
